# EUROPEAN PATENT SPECIFICATION

(11) **EP 3 005 186 B1**
(45) Date of publication and mention of the grant of the patent: **15.01.2020**
(21) Application number: 13815734.2
(22) Date of filing: 20.12.2013
(51) Int. Cl.: A61M 5/172

(54) **PUMP CONTROLLING DEVICE THAT OBTAINS PARAMETER VALUES FROM INSULIN PUMP FOR EXECUTING FUNCTIONS**
PUMPENSTEUERUNGSVORRICHTUNG ZUM ABRUFEN VON PARAMETERWERTE VON EINER INSULINPUMPE ZUM AUSFÜHREN VON FUNKTIONEN
DISPOSITIF DE COMMANDE DE POMPE QUI OBTIENT DES VALEURS DE PARAMÈTRES PROVENANT D'UNE POMPE À INSULINE ET PERMETTANT D'EXÉCUTER DES FONCTIONS

(30) Priority: 26.12.2012 US 201213726884
(43) Date of publication of application: 13.04.2016
(73) Proprietor: Roche Diabetes Care GmbH, 68305 Mannheim (DE); F. Hoffmann-La Roche AG, 4070 Basel (CH)
(72) Inventor: IMHOF, Erich, CH-3427 Utzenstorf (CH); KONRAD, Guido, 5608 Stetten AG (CH); LONG, James R., Fishers, Indiana 46038 (US); PASH, Phillip E., Indianapolis, Indiana 46219-3952 (US); REINKE, Robert E., Indianapolis, Indiana 46236 (US)
(74) Representative: Bittner, Thomas L.
(86) International application number: PCT/EP2013/077552
(87) International publication number: WO 2014/102159

(56) References cited:
- US-A1- 2011 154 237
- US-A1- 2011 320 049
- US-A1- 2012 277 716

## Description

### FIELD

The present disclosure relates to a pump controlling device and, more particularly, relates to a pump controlling device that obtains parameter values from an insulin pump for executing functions.

### BACKGROUND

Diabetes mellitus, often referred to as diabetes, is a chronic condition in which a person has elevated blood glucose levels that result from defects in the body's ability to produce and/or use insulin. Diabetes can be treated by injecting predetermined dosages of insulin to the patient to control the level of glucose in the bloodstream. For instance, some diabetes patients rely on an insulin pump to deliver the predetermined dosages to the patient.

The insulin pump can closely imitate a normally functioning pancreas by releasing multiple small doses of insulin each day into the body through an infusion set to regulate blood glucose levels. The dosage delivery rate of these small doses (i.e., the basal rate) can vary from user to user. Also, even for a particular user, the basal rate can change throughout the day, and the basal rate can depend upon various factors (e.g., the user's metabolism, physical health, stress levels, amount of exercise, etc.).

Insulin pumps can also deliver (either automatically or selectively) bolus doses of insulin. These bolus doses can be delivered before meals or snacks to compensate for the caloric intake. Also, bolus dosages can be delivered to correct high blood glucose levels. Moreover, the pump can be configured to deliver multiple types of bolus dosages (e.g., a "standard bolus," an "extended bolus," a "combination bolus/multiwave bolus," and a" super bolus"). These dosages can be adjusted according to the patient's particular physiology, eating habits, etc.

Many insulin pumps are programmable so that the basal and bolus dosages can be tailored to the particular user. Some pumps are also capable of communicating with a separate computing device and are compatible with software applications that may be executed on the computing device.

Document US 2011/0154237 A1 discloses a method of configuring a medical device, for example an insulin pump, through utilization of a computing device which includes a user interface, a processor and memory. A configuration file is transferred from the medical device to the computing device. At the computing device, a user can edit the configuration file. The amended configuration file is transferred back to the medical device. The method further includes a step of determining whether the configuration file contains a focal modified parameter, displaying the focal modified parameter, and prompting a manual reentry of the displayed focal modified parameter for confirmation by a user.

### SUMMARY

It is an object of the current invention to provide a safe and flexible pump controlling device or rather a system for pump controlling with such a pump controlling device. The objective is solved according to the subject matter of the independent claims.

A system for operating an insulin treatment according to claim 1, a pump controlling device according to claim 11, and a method according to claim 14 are disclosed.

A method of operating an insulin treatment system is disclosed. The insulin treatment system includes an insulin pump and a pump controlling device. The pump controlling device communicates with the insulin pump for obtaining parameter values for executing functions. The method includes receiving, by the pump controlling device, a request to execute a function that is included on the pump controlling device. The function is governed by a rule having a parameter, and the function is related to delivery of insulin by the insulin pump. Also, the method includes establishing communication between the pump controlling device and the insulin pump. Moreover, the method includes requesting, by the pump controlling device, a value for the parameter from the insulin pump and receiving, by the pump controlling device, the value for the parameter from the insulin pump. Furthermore, the method includes executing, by the pump controlling device, the function using the received value for the parameter.

In additional embodiments, the rule limits insulin delivery up to a maximum dosage, and the value for the parameter is the maximum insulin dosage amount. Also, in some embodiments, the rule limits bolus insulin delivery up to a maximum bolus dosage, and the value for the parameter is the maximum bolus dosage amount.

Moreover, in some embodiments, the rule limits insulin delivery time to a maximum duration, and the value for the parameter is the maximum duration. Additionally, in some embodiments, the rule limits bolus delivery time to a maximum bolus duration, and the value for the parameter is the maximum bolus duration.

Furthermore, in some embodiments, the rule limits a lag time before commencement of insulin delivery, and the value for the parameter is the maximum lag time. Additionally, in some embodiments, the rule limits a bolus lag time before commencement of bolus insulin delivery, and the value for the parameter is the maximum bolus lag time.

Still further, in some embodiments, the function and the rule are commonly included on the pump and the pump controlling device, and the value for the parameter is available to the pump controlling device only through requesting the value from the insulin pump and receiving the value from the insulin pump.

Moreover, the method can further include receiving, by the pump controlling device, a request to change the value for the parameter to a new value, establishing communication between the pump and the pump controlling device, transmitting the new value from the pump controlling device to the pump, and saving, by the insulin pump, the new value received from the pump controlling device.

Also, in some embodiments, the method can further include receiving a request that violates the rule, determining, by the pump controlling device that the request violates the rule, and further comprising outputting a message, by the pump controlling device, indicating that the request violates the rule. Outputting the message can include visually displaying the message on a display.

Also, a pump controlling device for remotely controlling an insulin pump is disclosed. The device includes a communication device that is operable to establish two way communication with the insulin pump. The device also includes a processor that is operable to receive a request to execute a function that is included on the pump controlling device. The function is governed by a rule having a parameter, and the function is related to delivery of insulin by the insulin pump. The processor is further operable to request, via the communication device, a value for the parameter from the insulin pump. The processor is also operable to receive the value for the parameter from the insulin pump, and the processor is operable to execute the function using the received value for the parameter.

Still further, a method of operating an insulin treatment system that includes an insulin pump and a pump controlling device is disclosed. The pump controlling device communicates with the insulin pump for obtaining parameter values for executing functions. The method includes receiving, by the pump controlling device, a request to execute a function related to delivery of a bolus insulin dosage function. The function is included on the pump and the pump controlling device, and the function is governed by a rule. The rule limits bolus insulin delivery up to a maximum bolus dosage. A value for the maximum bolus dosage is included on the pump. The method also includes establishing communication between the pump controlling device and the insulin pump. The method further includes requesting, by the pump controlling device, the value for the maximum bolus dosage from the insulin pump. Moreover, the method includes receiving, by the insulin pump, the request for the value for the maximum bolus dosage from the pump controlling device. Also, the method includes sending, by the insulin pump, the value for the maximum bolus dosage to the pump controlling device. Furthermore, the method includes receiving, by the pump controlling device, the value for the maximum bolus dosage from the insulin pump. Additionally, the method includes executing, by the pump controlling device, the function using the received value for the maximum bolus dosage.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The drawings described herein are for illustrative purposes only of selected embodiments and not all possible implementations, and are not intended to limit the scope of the present disclosure.
FIG. 1 is a schematic illustration of a diabetes treatment system according to various exemplary embodiments of the present disclosure;
FIG. 2 is an isometric view of an insulin pump and an infusion set that can be implemented in the system of FIG. 1 according to exemplary embodiments of the present disclosure;
FIG. 3 is a front view of a combination blood glucose meter and pump controlling device that can be implemented in the system of FIG. 1 according to exemplary embodiments of the present disclosure;
FIG. 4 is a flowchart representing exemplary methods of operating the diabetes treatment system of FIG. 1; and
FIG. 5 is a flowchart representing exemplary methods of delivering a bolus dosage of insulin using the diabetes treatment system of FIG. 1.

Corresponding reference numerals indicate corresponding parts throughout the several views of the drawings.

### DETAILED DESCRIPTION

Example embodiments will now be described more fully with reference to the accompanying drawings.

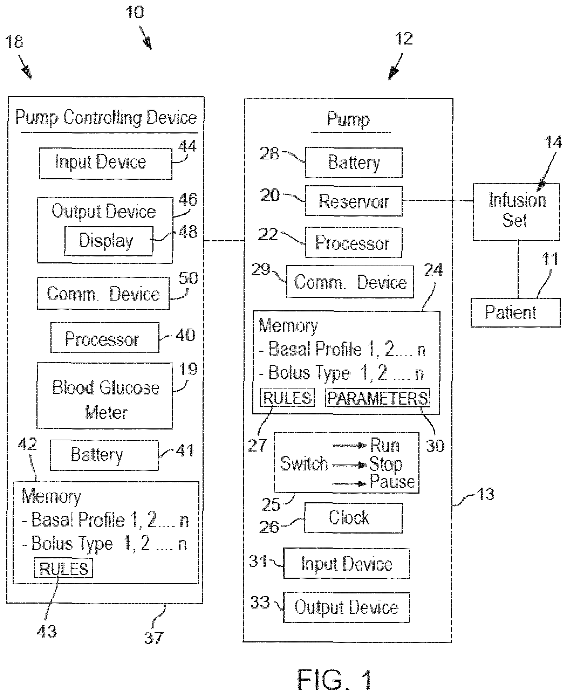

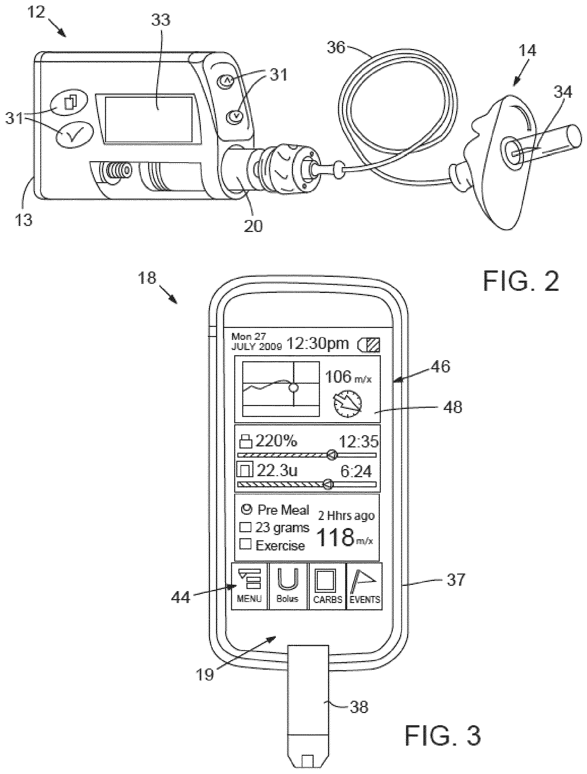

Referring initially to FIG. 1, a system 10 for delivering controlled dosages of insulin to a patient 11 is illustrated schematically. The system 10 can generally include an insulin pump 12, an infusion set 14, and a pump controlling device 18. Exemplary embodiments of the insulin pump 12 and infusion set 14 are illustrated in FIG. 2. Also, exemplary embodiments of the pump controlling device 18 are illustrated in FIG. 3. In the embodiments shown in FIG. 3, the pump controlling device 18 is embodied on a handheld or otherwise portable blood glucose meter 19; however, the pump controlling device 18 could be separate from a blood glucose meter in some embodiments.

Referring to FIGS. 1 and 2, the insulin pump 12 can incorporate various features of a known, wearable, and portable insulin pump. Thus, the insulin pump 12 can include a housing 13 (FIG. 2) that supports at least one reservoir 20 (i.e., insulin cartridge) which is prefilled or refillable. (The reservoir 20 is shown partially removed from the housing 13 in FIG. 2.) The reservoir 20 can selectively deliver insulin to the infusion set 14 as will be described in greater detail below.

The pump 12 can also include a processor 22 (i.e., controller) that includes programmed logic and/or other elements configured to control the start and stoppage of insulin delivery from the reservoir 20, the flow rate of the insulin, etc. The pump 12 can additionally include one or more memory devices 24 (FIG.1). The memory device 24 of the pump is preferably configured to store application programs and data and can be constructed of any suitable combination of volatile and/or nonvolatile memory. The memory device 24 can also store one or more predefined dosage schedules (i.e., dosage "profiles") that are tailored to the particular patient. In the embodiments illustrated in FIG. 1, the processor 22 includes a plurality of different basal dosage profiles (indicated as "Basal Profile: 1, 2...n"), and each of these profiles can dictate different basal dosage rates. The memory device 24 can also store one or more bolus dosage types (indicated as "Bolus Type: 1, 2...n"), which can represent any number of standard bolus dosages, extended bolus dosages, combination bolus/multiwave bolus dosages, super bolus dosages, etc. As will be discussed, the processor 22 is configured to access these profiles stored within the memory device 24 for controlling the amount of insulin delivered, the time of delivery, the rate of delivery, etc. It will be appreciated that the memory device 24 can store any number and type of dosage profile without departing from the scope of the present disclosure.

Moreover, the memory device 24 of the pump is preferably configured to store a RULES database 27, which stores one or more rules that govern respective functions of the pump 12. The memory device 24 is also configured to store a PARAMETERS database 30, which stores one or more parameters (i.e., values for variables) contained in corresponding rules within the database 27. For instance, one programmed function of the pump 12 can be a START BOLUS DELIVERY function. Thus, the RULES database 27 can include a rule that limits the maximum bolus dosage allowed in executing this function (e.g., Maximum Bolus Dosage = X). The "X" within that rule is a parameter (i.e., variable) with an unassigned value. However, the database 30 can include the value for X (i.e., the value for the parameter). Thus, as will be discussed, to execute the START BOLUS DELIVERY function, the processor 22 is configures to obtain the maximum bolus dosage rule from the database 27, and the processor 22 is also configured to obtain the value for the maximum bolus dosage from the database 30. These and other functions, rules, and parameters, will be discussed in greater detail below.

It will be appreciated that the RULES database 27 can be common to different pumps 22 used by different patients 11. However, the PARAMETERS database 30 can be tailored according to the particular patient 11 using the pump 12 such that the pump 12 performs according to the specific needs of the patient 11.

As shown in FIG. 1, the pump 12 can also comprise a switch, which is schematically illustrated and indicated at 25. The switch 25 is configured to be used for changing the operating state of the pump 12 between two or more operating states. In the embodiments illustrated in FIG. 1, there are three operating states of the pump, RUN, STOP, and PAUSE. In the RUN mode, the pump 12 is able to deliver insulin, in the STOP mode, the pump 12 is unable to deliver insulin, and in the PAUSE mode, the pump 12 is temporarily unable to deliver insulin (e.g., due to the reservoir 20 being empty, a failure detection etc.). It will be appreciated that the switch 25 can be realized as a substantially electrical switch (i.e., embodied as circuitry) as opposed to a mechanical switch with moving parts.

Also, the pump 12 can comprise a clock 26, which keeps track of the current date and time. By monitoring the clock 26, the processor 22 is configured to track when dosages are delivered. The memory device 24 is thus configured to save the dosage amount, the dosage type, the dosage date and time, and other data related to insulin dosages delivered by the pump 12 for future reference.

Moreover, the pump 12 preferably includes a power source, such as a battery 28, for providing power to the components of the pump 12. The battery 28 preferably includes a main battery that supplies power for normal operations of the pump 12, and the battery 28 more preferably includes a backup battery that supplies power for only essential operations of the pump 12 when the main battery fails. It will be appreciated that the pump 12 can include additional or alternative power sources (e.g., one or more capacitors, etc.) without departing from the scope of the present disclosure.

Additionally, the pump 12 can include one or more input devices 31 that can be used by the patient 11 for inputting commands directly to the pump 12. As shown in FIG. 2, the input devices 31 can include one or more buttons that the patient 11 can depress for inputting such commands; however, the input device 31 could include a touch-sensitive surface, a sliding switch, or other input device. The pump 12 can further include one or more output devices 33 that can output one or more messages (e.g., messages relating to dosages, etc.). In the embodiments of FIG. 2, the output device 33 includes a display screen for outputting the messages visually; however, the output device 33 could include a speaker for outputting the messages aurally. Moreover, in some embodiments, the output device can include a tactile, vibrating motor for outputting the messages in a tactile manner.

The pump 12 can further include a communications device 29. The communication device 29 is configured to establish communications between the pump 12 and the pump controlling device 18 as will be discussed in detail below. The communications device 29 can include a wireless transceiver (e.g., BLUETOOTH™ transceiver, etc.), and/or the communications device 29 can include a connector for connecting a wire between the pump 12 and the pump controlling device 18.

Furthermore, the infusion set 14 can be of a known type. Thus, the infusion set 14 can include a cannula 34 that is inserted subcutaneously into the patient 11 (i.e., the user, the person with diabetes, etc.). The infusion set 14 can also include a tube 36 that fluidly connects the cannula 34 to the reservoir 20 of the pump 12. As such, insulin can be delivered from the reservoir 20 and into the patient via the infusion set 14.

Referring now to FIGS. 1 and 3, embodiments of the pump controlling device 18 (from now on described mainly as "device 18") will be discussed in detail. The pump controlling device 18 can include a housing 37 that houses the components of the device 18. As shown in FIG. 1, the pump controlling device 18 can include a processor 40, which can include programmed logic and/or other elements so that the processor is configured to control the device 18 and to send control commands to the pump 12.

The device 18 can also include a memory device 42, which is configured to store application programs and data and can be constructed of any suitable combination of volatile and/or nonvolatile memory. As shown in FIG. 1, the memory device 42 can include basal and bolus profiles, which can be the same as those included on the memory device 24 of the pump 12. The memory device 42 can also include a RULES database 43, which can be the same as the database 27 included on the memory device 24 of the pump 12. However, it is noted that the memory device 24 does not include the PARAMETERS database 30 included on the memory device 24 of the pump 12. Thus, the pump controlling device 18 does not include the values of the parameters that govern pumping functions, and as will be discussed in greater detail below, the pump controlling device 18 can request and receive values of the parameters from the database 30 of the pump 12 in order to execute certain functions.

Moreover, the device 18 can include a battery 41 or other power source that supplies power to the components of the device 18. Also, the device 18 can include one or more input devices 44 with which the patient 11 can input commands. The input devices 44 can include buttons, switches, a touch sensitive surface, or any other suitable device. The device 18 can further include one or more output devices 46 that output information relating to operations of the system 10. The output devices 46 can be of any suitable type, such as a display 48 that outputs information visually, a speaker that outputs audible information, a vibrating motor that outputs tactile information, etc. In the embodiments of FIG. 3, the device 18 includes the display 48, and the display 48 includes one or more touch-sensitive areas, such that the display 48 can function as both an input device 44 and an output device 46. Also, as shown in FIG. 3, the display 48 is configured to display various information, such as the current date and time, graphical information about insulin dosages, etc.. Furthermore, the display 48 is configured to display user selectable options for allowing the patient 11 to enter bolus information (labeled "Bolus" in FIG. 3), carbohydrate information (labeled "Carbs" in FIG. 3), or other information related to meals, exercise, periods of stress, physiological events such as menstruation, etc. (labeled "Events" in FIG. 3).

Also, as mentioned above, the pump controlling device 18 preferably comprises a blood glucose (bG) meter 19. The meter 19 can be of a known type configured to detect the current (i.e., actual) blood glucose level of the patient 11. More specifically, the patient 11 can apply blood to a test strip 38 (FIG. 3), and the meter 19 is configured to receive the strip 38 and to detect the amount of glucose in the blood thereon. This information can be useful for calculating an appropriate bolus dosage or for other purposes. Also, this information can be stored in the memory device 42 in a suitable database for future analysis.

The blood glucose readings can also be associated or otherwise stored with other information in the memory device 42. For instance, the memory device 42 is configured to store the blood glucose readings with other health related information of the particular patient 11. More specifically, the memory device 42 is configured to store bolus recommendation history records as well as the inputs for the recommendations, such as carbohydrate intake and blood glucose level. The memory device 42 is preferably further configured to store health, carbohydrate, and blood-glucose-related variables (e.g., insulin sensitivities of the patient 11 for particular time segments of particular days of the week, etc.).

The device 18 can further include a communication device 50, such as a wireless transceiver (e.g., a BLUETOOTH™ transceiver, etc.) or a connector for connecting a wire. Thus, the communication device 50 of the pump controlling device 18 is configured to selectively communicate with the communication device 29 of the insulin pump 12 wirelessly and/or via a hardwire connection. As will be discussed, the communication devices 50, 29 is preferably configured to provide two-way communication between the pump controlling device 18 and the insulin pump 12.

Thus, the processor 40 is configured to run software stored in the memory device 42. Also, various input commands can be provided from the patient 11 via the input device 44 (e.g., the touch-sensitive surface of the display 48) for performing various functions. For instance, the processor 40 can be configured to calculate a recommended meal bolus, a recommended correction bolus, a recommended total bolus, and/or a suggested carbohydrate amount in this manner. In particular, the processor 40 is configured to cause the communication device 50 to transmit various control commands to the pump 12. The pump controlling device 18 is preferably configured to send a variety of control commands, such as a START BOLUS DELIVERY, STOP PUMP, and other commands. The insulin amount, dosage time, insulin flow rate, etc. can also be specified in this command.

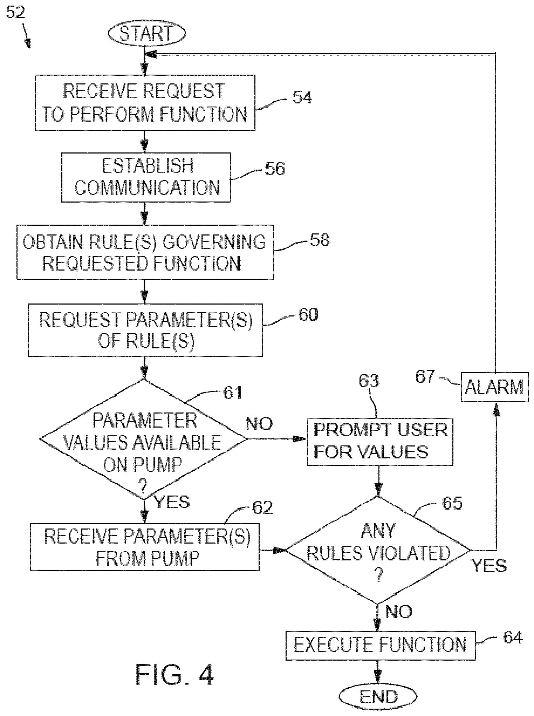

Referring now to FIG. 4, exemplary embodiments of a method 52 of operating the system 10 will be discussed. It will be appreciated that this method 52 can be used when the patient 11 uses the pump controlling device 18 to control the pump 12.

As shown, the method 52 can begin in block 54, wherein the device 18 receives a request from the patient 11 to perform a function. The patient 11 can input a request using the input device 44 of the pump control device 18. Block 54 can include any suitable request. For instance, the patient 11 can use the device 18 to request delivery of a bolus dosage at a specified level (e.g., request delivery of 0.1 insulin units).

Then, in block 56, the pump control device 18 is configured to establish communication with the pump 12. For instance, the communication device 50 of the device 18 is configured to establish communication with the communication device 29 of the pump 12 to thereby pair the device 18 and the pump 12.

Next, in block 58, the pump control device 18 is configured to obtain the rules that govern the function requested in block 54. In the example mentioned above (where the patient 11 requests delivery of 0.1 insulin units for a bolus dosage), block 58 can include the pump control device 18 configured to obtain rules that govern bolus dosages (e.g., a rule governing a maximum bolus dosage). The rules can be obtained from the database 43 that is local to the device 18, or the rules can be obtained from the database 27 included on the pump 12.

Subsequently, in block 60, the device 18 is configured to request parameter values for the rules obtained in block 58. In particular in the bolus dosage example mentioned above, block 60 can include that the device 18 is configured to request a value for the maximum bolus dosage. This request can be sent to the pump 12 via communications between the communication devices 50, 29.

Then, in decision block 61, it can be determined whether the parameter values are available in the database 30 of the pump 12. If the parameter values are available (decision block 61 answered affirmatively), the pump 12 is configured to send the parameter values to the device 18 and, in block 62, the pump control device 18 is configured to receive the parameter values from the pump 12. However, if the parameter values are unavailable (e.g., because they have not been assigned yet, etc.) (decision block 61 answered negatively), the display 48 of the device 18 can prompt the patient 11 to enter the necessary parameter values in block 63. Once entered, the device 18 is configured to transmit the parameter values to the pump 12 for storage in the database 30.

Then, after block 62 or block 63, the method 52 can continue in decision block 65, wherein it is determined whether any of the rules (obtained in block 58) have been violated by the request of block 54. The processor 40 of the device 18 is configured to make this determination locally, or the device 18 is configured to communicate with the pump 12 to make this determination. Continuing with the example discussed above, the processor 40 is configured to find violation (block 65 answered affirmatively) if the requested 0.1 insulin units exceeds the maximum bolus dosage limit received in block 62 or set in block 63. On the other hand, the processor 40 is configured to determine that there is no violation by the requested 0.1 insulin units if the maximum bolus dosage limit is equal to or less than 0.1 insulin units.

If there has been a violation (decision block 65 answered affirmatively), then in block 67, the output device 46 of the device 18 is configured to output an alarm to notify the patient 11 of the violation. This alarm can be a visual message displayed on the display 48, an audible alarm, a tactile alarm, or any other type of alarm. However, if no rule has been violated (decision block 65 answered negatively), then in block 64, the pump control device 18 is configured to execute the function requested in block 54.

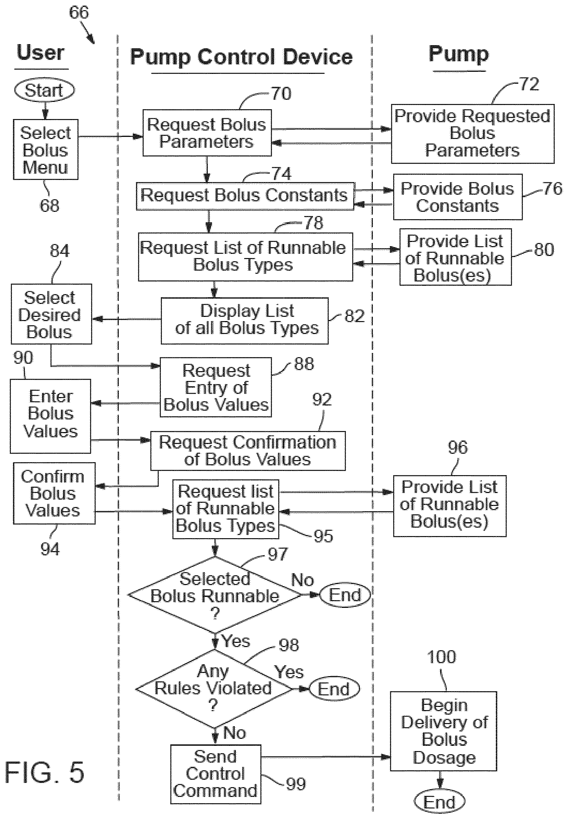

Referring now to FIG. 5, more detailed embodiments of a system 10 and its operation and how the system 10 is configured to operate a method 66 are illustrated. In this example, the patient 11 desires for the pump 12 to begin delivery of a bolus insulin dosage. The patient 11 uses the pump control device 18 to send control commands to the pump 12 for executing this function as will be discussed. It is assumed that the device 18 and the pump 12 are paired and the devices 29, 50 are providing two-way communication therebetween.

The method 66 can begin in block 68, wherein the patient 11 selects a bolus menu on the display 48 of the pump controlling device 18. The pump controlling device 18 can begin the process of displaying the available types of bolus dosages that can be delivered by the pump 12 back to the patient 11. However, the pump controlling device 18, in block 70, can first request the bolus parameter values from the pump 12. In some embodiments, the pump controlling device 18 can request values for the maximum bolus amount limit (i.e., the maximum bolus dosage allowed), the duration of the bolus dosage (i.e., the length of time for delivery of the bolus dosage), the lag time for the bolus dosage (i.e., the amount of time before commencing delivery of the bolus dosage), or other parameter values.

Then, in block 72, the pump 12 can reply to the request of block 70. Specifically, the pump 12 can obtain the requested parameter values stored in the database 30 and send back those parameter values via the communication device 29.

Next, in block 74, the pump controlling device 18 can request from the pump 12 bolus constants that are also included in the rules governing the desired function. In some embodiments, the pump controlling device 18 can request constants, such as a bolus amount resolution, a bolus amount range threshold, or other constants. Then, in block 76, the pump 12 can obtain the requested constants from the memory device 24 and send back the obtained constants.

Additionally, in block 78, the pump controlling device 18 can request from the pump 12 a list of all of the bolus dosage types that can be currently run. The pump 12, in turn, can provide such a list of runnable bolus dosage types back to the device 18 in block 80. In some embodiments, the processor 22 of the pump 12 can calculate or otherwise determine the bolus types that are runnable based on preprogrammed rules.

Then, in block 82, the display 48 of the device 18 can display the list of bolus types that are available. This list can include standard bolus types and customized bolus types. Next, in block 84, the patient 11 can select the desired bolus type from the list displayed in block 82. In other embodiments of block 82, the display 48 can display all of the bolus types, but those that are currently unrunnable can be distinguished from those that are currently runnable. For instance, the bolus types that are currently unrunnable can be displayed using respective unselectable icons (e.g., "greyed out" icons) while the runnable bolus types can be displayed as selectable icons.

Next, in block 88, the pump controlling device 18 can prompt the patient 11 to enter desired bolus dosage values. These values can relate to the number of bolus insulin units necessary for lowering the patient's current high blood glucose level, the duration of the bolus dosage, the lag time for the bolus dosage, or other values. Once prompted, the patient 11, in block 90, can enter the bolus values requested in block 88 using the input device 44. The pump control device 18, in block 92, can request confirmation of the bolus values entered in block 90, and in block 94, the patient 11 can provide confirmation.

Subsequently, in blocks 95, 96, and 97, the pump controlling device 18 can re-check with the pump 12 that the bolus type selected in block 84 is still runnable. Specifically, in block 95, the pump controlling device 18 can request from the pump 12 a list of all of the bolus dosage types that can be currently run (similar to block 78). Then, in block 96, the pump 12 can provide the list of currently runnable bolus dosage types (similar to block 80). In block 97, the pump controlling device 18 can determine whether the bolus type selected in block 84 is currently runnable (as dictated by the pump 12 in block 96). If the selected bolus type is not runnable (decision block 97 answered negatively), then the method 66 can end as shown in FIG. 5. Alternatively, the display 48 could inform the patient 11 that the selected bolus is currently unrunnable, and the method 66 could loop back to block 84 to allow the patient 11 to select another bolus type. If selected bolus type is runnable (decision block 97 answered affirmatively), then the method 66 can continue in block 98.

In block 98, the pump controlling device 18 can determine whether any rules are violated by the bolus values entered in block 90. This determination can be similar to block 65 of FIG. 4. Specifically, the processor 40 can compare the bolus values entered in block 90 to the bolus parameters received in block 70 to see whether any rules have been violated. For instance, if the bolus values entered by the patient 11 in block 90 exceed the set maximum bolus dosage limits received in block 70, then the processor 40 can determine that there is a rule violation. Similar comparisons can be made for other values entered in block 90 (e.g., the duration of the bolus dosage, the lag time for the bolus dosage, etc.).

If there is a violation (block 98 answered affirmatively), then the method 66 can end as shown in FIG. 5. Alternatively, the display 48 could inform the patient 11 that the values entered in block 90 are unacceptable or could display another error message, and the method 66 could loop back to block 88 to request entry of other values.

On the other hand, if there is no violation (block 98 answered negatively), then block 99 can follow. In block 99, the pump controlling device 18 can send a control command to the pump 12 to initiate delivery of the selected bolus dosage. As a result, the pump 12 can begin delivery of the selected bolus dosage as shown in block 100.

Accordingly, the system 10 and its methods 52, 66 of use can ensure that the pump control device 18 and the pump 12 communicate effectively and accurately. For instance, because the parameter values are stored only in the database 30 of the pump 12, and the pump control device 18 must request and obtain parameter values therefrom, there are unlikely to be conflicting parameter values. Stated differently, even though both the pump control device 18 and the pump 12 can be "aware" of the rules included on the respective databases 43, 27, the parameter values are only stored in the database 30 of the pump 12; therefore, the system 10 can operate more effectively and accurately.

The techniques described herein may be implemented by one or more computer programs executed by one or more processors. The computer programs include processor-executable instructions that are stored on a non-transitory tangible computer readable medium. The computer programs may also include stored data. Non-limiting examples of the non-transitory tangible computer readable medium are nonvolatile memory, magnetic storage, and optical storage.

Some portions of the above description present the techniques described herein in terms of algorithms and symbolic representations of operations on information. These algorithmic descriptions and representations are the means used by those skilled in the data processing arts to most effectively convey the substance of their work to others skilled in the art. These operations, while described functionally or logically, are understood to be implemented by computer programs. Furthermore, it has also proven convenient at times to refer to these arrangements of operations as modules or by functional names, without loss of generality.

Unless specifically stated otherwise as apparent from the above discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices.

Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems.

The present disclosure also relates to an apparatus for performing the operations herein. This apparatus may be specially constructed for the required purposes, or it may comprise a general-purpose computer selectively activated or reconfigured by a computer program stored on a computer readable medium that can be accessed by the computer. Such a computer program may be stored in a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The algorithms and operations presented herein are not inherently related to any particular computer or other apparatus. Various general-purpose systems may also be used with programs in accordance with the teachings herein, or it may prove convenient to construct more specialized apparatuses to perform the required method steps. The required structure for a variety of these systems will be apparent to those of skill in the art, along with equivalent variations. In addition, the present disclosure is not described with reference to any particular programming language. It is appreciated that a variety of programming languages may be used to implement the teachings of the present disclosure as described herein.

The present disclosure is well suited to a wide variety of computer network systems over numerous topologies. Within this field, the configuration and management of large networks comprise storage devices and computers that are communicatively coupled to dissimilar computers and storage devices over a network, such as the Internet.

## Claims

1. A system (10) for operating an insulin treatment, comprising an insulin pump (12) and a pump controlling device (18), wherein the pump controlling device (18) is configured to communicate with the insulin pump (12) for obtaining parameter values for executing functions, wherein the pump controlling device (18) is configured to receive a request to execute a function and wherein a processor (40) of the pump controlling device (18) is configured to perform the function, a memory device (42) of the pump controlling device (18) is configured to store software which implements the function being governed by a rule having a parameter, the function being related to delivery of insulin by the insulin pump (12); wherein the rule is stored in the memory device (42) of the pump controlling device (18) or wherein the rule is stored in the memory device (42) of the pump controlling device (18) and in a memory device (24) of the insulin pump (12); wherein the insulin pump (12) and the pump controlling device (18) comprise each a communication device (29, 50) which are configured to establish a communication between the pump controlling device (18) and the insulin pump (12); wherein the pump controlling device (18) and the insulin pump (12) are configured so that
- the pump controlling device (18) requests a value for the parameter from the memory device (24) of the insulin pump (12);
- the pump controlling device (18) receives the value for the parameter from the insulin pump (12);
- the pump controlling device (18) determines, by the processor (40), whether the rule is violated by the request based on the value for the parameter; and
- the pump controlling device (18) executes the function using the received value for the parameter, if the rule is not violated.

2. The system of claim 1, wherein the rule limits insulin delivery up to a maximum dosage, and wherein the value for the parameter is the maximum insulin dosage amount.

3. The system of claim 2, wherein the processor (40) is configured so that the rule limits bolus insulin delivery up to a maximum bolus dosage, and wherein the value for the parameter is the maximum bolus dosage amount.

4. The system of claim 1, 2 or 3, wherein the processor (40) is configured so that the rule limits insulin delivery time to a maximum duration, and wherein the value for the parameter is the maximum duration.

5. The system of claim 4, wherein the processor (40) is configured so that the rule limits bolus insulin delivery time to a maximum bolus duration, and wherein the value for the parameter is the maximum bolus duration.

6. The system of any of the preceding claims, wherein the processor (40) is configured so that the rule limits a lag time before commencement of insulin delivery, and wherein the value for the parameter is the maximum lag time.

7. The system of claim 6, wherein the processor (40) is configured so that the rule limits a bolus lag time before commencement of bolus insulin delivery, and wherein the value for the parameter is the maximum bolus lag time.

8. The system of any of any of the preceding claims, wherein the pump controlling device (18) is configured to receive a request to change the value for the parameter to a new value, establishing communication between the insulin pump (12) and the pump controlling device (18) via the communication devices (29, 50), transmitting the new value from the pump controlling (18) device to the insulin pump (12), and wherein only the memory device (24) of the insulin pump (12) is configured to save the new value received from the pump controlling device (18).

9. The system according to any of the preceding claims, wherein the pump controlling device (18) is configured to receive a request that violates the rule, , and wherein the pump controlling device (18) is configured to outputting a message, indicating that the request violates the rule.

10. The system of claim 9, wherein a display (48) of an output device (46) of the pump controlling device (18) is configured to output the message by visually displaying the message.

11. A pump controlling device (18) for remotely controlling an insulin pump (12) comprising:
- a communication device (50) that is operable to establish two way communication with the insulin pump (12);
- a memory device (42);
- a processor (40) that is operable to receive a request to execute a function that is included on the pump controlling device (18), the function being governed by a rule having a parameter, wherein the rule is stored in the memory device (42) of the pump controlling device (18) or wherein the rule is stored in the memory device (42) of the pump controlling device (18) and in a memory device (24) of the insulin pump (12), the function being related to delivery of insulin by the insulin pump, the processor (40) further operable to request, via the communication device (50), a value for the parameter from the insulin pump (12), the processor (40) also operable to receive the value for the parameter from the insulin pump (12), the processor (40) also operable to determine whether the rule is violated by the request based on the value for the parameter, and the processor (40) also operable to execute the function using the received value for the parameter, if the request is not violated.

12. The pump controlling device of claim 11, wherein the rule limits insulin delivery time to a maximum duration, and wherein the value for the parameter is the maximum duration, in particular wherein the rule limits a lag time before commencement of insulin delivery, and wherein the value for the parameter is the maximum lag time.

13. The pump controlling device of claim 11 or 12, further comprising an output device, and wherein the output device is operable to output a message indicating that the request violates the rule, in particular wherein the output device is a display that is operable to visually display the message.

14. A method for operating an insulin treatment the system (10) which comprises an insulin pump (12) and a pump controlling device (18), wherein the pump controlling device (18) is configured to communicate with the insulin pump (12) for obtaining parameter values for executing functions, the method comprising:
- receiving, by the pump controlling device (18), a request to execute a function, wherein a processor (40) of the pump controlling device (18) is configured to perform this function, wherein a memory device (42) of the pump controlling device (18) is configured to store software which implements the function being governed by a rule having a parameter, the function being related to delivery of insulin by the insulin pump (12); wherein the rule is stored in the memory device (42) of the pump controlling device (18) or wherein the rule is stored in the memory device (42) of the pump controlling device (18) and in a memory device (24) of the insulin pump (12);
- establishing communication between the insulin pump (12) and the pump controlling device (18);
- requesting, by the pump controlling device (18), a value for the parameter from the memory device (24) of the insulin pump (12);
- receiving, by the pump controlling device (18), the value for the parameter from the insulin pump (12);
- determining, by the processor (40) of the pump controlling device (18), whether the rule is violated by the request based on the value for the parameter; and
- executing, by the pump controlling device (18), the function using the received value for the parameter, if the rule is not violated.

## Patentansprüche

1. System (10) zum Ausführen einer Insulinbehandlung, umfassend eine Insulinpumpe (12) und eine Pumpensteuervorrichtung (18), wobei die Pumpensteuervorrichtung (18) zum Kommunizieren mit der Insulinpumpe (12) zum Erhalten von Parameterwerten zum Ausführen von Funktionen konfiguriert ist, wobei die Pumpensteuervorrichtung (18) zum Empfangen einer Anfrage konfiguriert ist, eine Funktion auszuführen, und wobei ein Prozessor (40) der Pumpensteuervorrichtung (18) zum Ausführen der Funktionen konfiguriert ist, eine Speichervorrichtung (42) der Pumpensteuervorrichtung (18) zum Speichern von Software konfiguriert ist, die die Funktion implementiert, die durch eine einen Parameter aufweisende Regel bestimmt wird, wobei die Funktion mit der Verabreichung von Insulin durch die Insulinpumpe (12) verbunden ist; wobei die Regel in der Speichervorrichtung (42) der Pumpensteuervorrichtung (18) gespeichert ist oder wobei die Regel in der Speichervorrichtung (42) der Pumpensteuervorrichtung (18) und in einer Speichervorrichtung (24) der Insulinpumpe (12) gespeichert ist; wobei die Insulinpumpe (12) und die Pumpensteuervorrichtung (18) jeweils eine Kommunikationsvorrichtung (29, 50) umfassen, die konfiguriert ist, eine Kommunikation zwischen der Pumpensteuervorrichtung (18) und der Insulinpumpe (12) einzurichten; wobei die Pumpensteuervorrichtung (18) und die Insulinpumpe (12) so konfiguriert sind, dass
- die Pumpensteuervorrichtung (18) einen Wert für den Parameter von der Speichervorrichtung (24) der Insulinpumpe (12) anfordert;
- die Pumpensteuervorrichtung (18) den Wert für den Parameter von der Insulinpumpe (12) empfängt;
- die Pumpensteuervorrichtung (18), durch den Prozessor (40), bestimmt, ob die Regel durch die Anforderung auf der Basis des Werts für den Parameter verletzt wird; und
- die Pumpensteuervorrichtung (18) die Funktion unter Anwendung des für den Parameter empfangenden Werts ausführt, wenn die Regel nicht verletzt wird.

2. System nach Anspruch 1, wobei die Regel die Insulinverabreichung bis zu einer maximalen Dosierung beschränkt und wobei der Wert für den Parameter die maximale Insulindosiermenge ist.

3. System nach Anspruch 2, wobei der Prozessor (40) so konfiguriert ist, dass die Regel die Bolusinsulinverabreichung bis zu einer maximalen Bolusdosierung beschränkt und wobei der Wert für den Parameter die maximale Bolusdosiermenge ist.

4. System nach Anspruch 1, 2 oder 3, wobei der Prozessor (40) so konfiguriert ist, dass die Regel die Insulinverabreichungszeit auf eine maximale Dauer beschränkt und wobei der Wert für den Parameter die maximale Dauer ist.

5. System nach Anspruch 4, wobei der Prozessor (40) so konfiguriert ist, dass die Regel die Bolusinsulinverabreichungszeit auf eine maximale Bolusdauer beschränkt und wobei der Wert für den Parameter die maximale Bolusdauer ist.

6. System nach einem der vorhergehenden Ansprüche, wobei der Prozessor (40) so konfiguriert ist, dass die Regel eine Verzögerungszeit vor Beginn der Insulinverabreichung beschränkt und wobei der Wert für den Parameter die maximale Verzögerungszeit ist.

7. System nach Anspruch 6, wobei der Prozessor (40) so konfiguriert ist, dass die Regel eine Bolusverzögerungszeit vor Beginn der Bolusinsulinverabreichung beschränkt und wobei der Wert für den Parameter die maximale Bolusverzögerungszeit ist.

8. System nach einem der vorhergehenden Ansprüche, wobei die Pumpensteuervorrichtung (18) konfiguriert ist, eine Anfrage, den Wert für den Parameter zu einem neuen Wert zu ändern, zu empfangen, die Kommunikation zwischen der Insulinpumpe (12) und der Pumpensteuervorrichtung (18) über die Kommunikationsvorrichtungen (29,50) einzurichten, den neuen Wert von der Pumpensteuervorrichtung (18) zu der Insulinpumpe (12) zu senden, und wobei nur die Speichervorrichtung (24) der Insulinpumpe (12) zum Speichern des neuen Werts, der von der Pumpensteuervorrichtung (18) empfangen worden ist, konfiguriert ist.

9. System nach einem der vorhergehenden Ansprüche, wobei die Pumpensteuervorrichtung (18) zum Aufnehmen einer Anfrage konfiguriert ist, die die Regel verletzt, und wobei die Pumpensteuervorrichtung (18) zum Ausgeben einer Meldung konfiguriert ist, die darauf hinweist, dass die Anfrage die Regel verletzt.

10. System nach Anspruch 9, wobei eine Anzeige (48) einer Ausgabevorrichtung (46) der Pumpensteuervorrichtung (18) zum Ausgeben der Meldung durch visuelles Anzeigen der Meldung konfiguriert ist.

11. Pumpensteuervorrichtung (18) zum Fernsteuern einer Insulinpumpe (12), umfassend:
- eine Kommunikationsvorrichtung (50) die funktionsfähig ist, Zweiwegkommunikation mit der insulinpumpe (12) einzurichten;
- eine Speichervorrichtung (42);
- einen Prozessor (40), der funktionsfähig ist, eine Anfrage zum empfangen, eine Funktion auszuführen, die in der Pumpensteuervorrichtung (18) enthalten ist, wobei die Funktion durch eine Regel reguliert ist, die einen Parameter aufweist, wobei die Regel in der Speichervorrichtung (42) der Pumpensteuervorrichtung (18) gespeichert ist oder wobei die Regel in der Speichervorrichtung (42) der Pumpensteuervorrichtung (18) und in einer Speichervorrichtung (24) der Insulinpumpe (12) gespeichert ist, wobei die Funktion mit der Verabreichung von Insulin durch die Insulinpumpe verbunden ist, wobei der Prozessor (40) ferner funktionsfähig ist, über die Kommunikationsvorrichtung (50) einen Wert für den Parameter von der Insulinpumpe (12) anzufordern, wobei der Prozessor (40) auch funktionsfähig ist, den Wert für den Parameter von der Insulinpumpe (12) zu empfangen, der Prozessor (40) auch funktionsfähig ist, zu bestimmen, ob die Regel durch die Anfrage auf der Basis des Werts für den Parameter verletzt wird und der Prozessor (40) auch funktionsfähig ist, die Funktion unter Anwendung des für den Parameter empfangenden Werts auszuführen, wenn die Anfrage nicht verletzt wird.

12. Pumpensteuervorrichtung nach Anspruch 11, wobei die Regel die Insulinverabreichung auf eine maximale Dauer beschränkt und wobei der Wert für den Parameter die maximale Dauer ist, wobei insbesondere die Regel eine Verzögerungszeit vor Beginn der Insulinverabreichung beschränkt und wobei der Wert für den Parameter die maximale Verzögerungszeit ist.

13. Pumpensteuervorrichtung nach Anspruch 11 oder 12, ferner eine Ausgabevorrichtung umfassend und wobei die Ausgabevorrichtung funktionsfähig ist, eine Meldung auszugeben, die anzeigt, dass die Anforderung die Regel verletzt, wobei insbesondere die Ausgabevorrichtung eine Anzeige ist, die funktionsfähig ist, die Meldung visuell anzuzeigen.

14. Verfahren zum Ausführen eines Insulinbehandlungssystems (10), das eine Insulinpumpe (12) und eine Pumpensteuervorrichtung (18) umfasst, wobei die Pumpensteuervorrichtung (18) zum Kommunizieren mit der Insulinpumpe (12) zum Erhalten von Parameterwerten zum Ausführen von Funktionen konfiguriert ist, wobei das Verfahren umfasst:
- Empfangen, durch die Pumpensteuervorrichtung (18), einer Anfrage, eine Funktion auszuführen, wobei ein Prozessor (40) der Pumpensteuervorrichtung (18) zum Ausführen dieser Funktion konfiguriert ist, wobei eine Speichervorrichtung (42) der Pumpensteuervorrichtung (18) zum Speichern von Software konfiguriert ist, die die Funktion ausführt, die durch eine Regel bestimmt wird, die einen Parameter aufweist, wobei die Funktion mit der Verabreichung von Insulin durch die Insulinpumpe (12) verbunden ist; wobei die Regel in der Speichervorrichtung (42) der Pumpensteuervorrichtung (18) gespeichert ist oder wobei die Regel in der Speichervorrichtung (42) der Pumpensteuervorrichtung (18) und in einer Speichervorrichtung (24) der Insulinpumpe (12) gespeichert ist;
- Herstellen von Kommunikation zwischen der Insulinpumpe (12) und der Pumpensteuervorrichtung (18);
- Anfordern, durch die Pumpensteuervorrichtung (18), eines Werts für den Parameter von der Speichervorrichtung (24) der Insulinpumpe (12);
- Empfangen, durch die Pumpensteuervorrichtung (18), des Werts für den Parameter von der Insulinpumpe (12);
- Bestimmen, durch den Prozessor (40) der Pumpensteuervorrichtung (18), ob die Regel durch die Anforderung auf der Basis des Werts für den Parameter verletzt wird; und
- Ausführen, durch die Pumpensteuervorrichtung (18), der Funktion unter Anwendung des für den Parameter empfangenen Werts, wenn die Regel nicht verletzt wird.

## Revendications

1. Système (10) pour mettre en œuvre un traitement par insuline, comprenant une pompe à insuline (12) et un dispositif de commande de pompe (18), dans lequel le dispositif de commande de pompe (18) est configuré pour communiquer avec la pompe à insuline (12) pour obtenir des valeurs de paramètre pour exécuter des fonctions, dans lequel le dispositif de commande de pompe (18) est configuré pour recevoir une requête d'exécution d'une fonction et dans lequel un processeur (40) du dispositif de commande de pompe (18) est configuré pour réaliser la fonction, un dispositif mémoire (42) du dispositif de commande de pompe (18) est configuré pour stocker un logiciel qui met en œuvre la fonction étant gouvernée par une règle ayant un paramètre, la fonction étant liée à l'administration d'insuline par la pompe à insuline (12) ; dans lequel la règle est stockée dans le dispositif mémoire (42) du dispositif de commande de pompe (18) ou dans lequel la règle est stockée dans le dispositif mémoire (42) du dispositif de commande de pompe (18) et dans un dispositif mémoire (24) de la pompe à insuline (12) ; dans lequel la pompe à insuline (12) et le dispositif de commande de pompe (18) comprennent chacun un dispositif de communication (29, 50) qui est configuré pour établir une communication entre le dispositif de commande de pompe (18) et la pompe à insuline (12); dans lequel le dispositif de commande de pompe (18) et la pompe à insuline (12) sont configurés de manière à ce que
- le dispositif de commande de pompe (18) envoie une requête d'une valeur pour le paramètre au dispositif mémoire (24) de la pompe à insuline (12) ;
- le dispositif de commande de pompe (18) reçoive la valeur pour le paramètre de la pompe à insuline (12) ;
- le dispositif de commande pompe (18) détermine, par le processeur (40), si la règle est violée par la requête sur la base de la valeur pour le paramètre ; et
- le dispositif de commande de pompe (18) exécute la fonction en utilisant la valeur reçue pour le paramètre, si la règle n'est pas violée.

2. Système selon la revendication 1, dans lequel la règle limite l'administration d'insuline à une dose maximale, et dans lequel la valeur du paramètre est la quantité de dose d'insuline maximale.

3. Système selon la revendication 2, dans lequel le processeur (40) est configuré de manière à ce que la règle limite l'administration de bolus d'insuline à une dose de bolus maximale, et dans lequel la valeur pour le paramètre est la quantité de dose de bolus maximale.

4. Système selon la revendication 1, 2 ou 3, dans lequel le processeur (40) est configuré de manière à ce que la règle limite la durée d'administration d'insuline à une durée maximale, et dans lequel la valeur du paramètre est la durée maximale.

5. Système selon la revendication 4, dans lequel le processeur (40) est configuré de manière à ce que la règle limite la durée d'administration de bolus d'insuline à une durée de bolus maximale, et dans lequel la valeur du paramètre est la durée de bolus maximale.

6. Système selon l'une quelconque des revendications précédentes, dans lequel le processeur (40) est configuré de manière à ce que la règle limite un décalage avant le début de l'administration d'insuline et dans lequel la valeur pour le paramètre est le décalage maximal.

7. Système selon la revendication 6, dans lequel le processeur (40) est configuré de manière à ce que la règle limite un décalage de bolus avant le début de l'administration du bolus d'insuline, et dans lequel la valeur du paramètre est le décalage de bolus maximal.

8. Système selon l'une quelconque des revendications précédentes, dans lequel le dispositif de commande de pompe (18) est configuré pour recevoir une requête de changement de la valeur du paramètre pour une nouvelle valeur, établissant une communication entre la pompe à insuline (12) et le dispositif de commande de pompe (18) via les dispositifs de communication (29, 50), transmettant la nouvelle valeur du dispositif de commande de pompe (18) à la pompe à insuline (12), et dans lequel l'unique dispositif mémoire (24) de la pompe à insuline (12) est configuré pour sauvegarder la nouvelle valeur reçue du dispositif de commande de pompe (18).

9. Système selon l'une quelconque des revendications précédentes, dans lequel le dispositif de commande de pompe (18) est configuré pour recevoir une requête qui viole la règle, et dans lequel le dispositif de commande de pompe (18) est configuré pour produire un message, indiquant que la requête viole la règle.

10. Dispositif selon la revendication 9, dans lequel un affichage (48) d'un dispositif d'émission (46) du dispositif de commande de pompe (18) est configuré pour produire le message par affichage visuel du message.

11. Dispositif de commande de pompe (18) pour télécommander une pompe à insuline (12) comprenant :
- un dispositif de communication (50) qui peut fonctionner pour établir une communication bidirectionnelle avec la pompe à insuline (12) ;
- un dispositif mémoire (42) ;
- un processeur (40) qui peut fonctionner pour recevoir une requête pour exécuter une fonction qui est incluse sur le dispositif de commande de pompe (18), la fonction étant gouvernée par une règle ayant un paramètre, dans lequel la règle est stockée dans le dispositif mémoire (42) du dispositif de commande de pompe (18) ou dans lequel la règle est stockée dans le dispositif mémoire (42) du dispositif de commande de pompe (18) et dans un dispositif mémoire (24) de la pompe à insuline (12), la fonction étant liée à l'administration d'insuline par la pompe à insuline, le processeur (40) pouvant en outre fonctionner pour envoyer une requête, via le dispositif de communication (50), d'une valeur pour le paramètre de la pompe à insuline (12), le processeur (40) pouvant aussi fonctionner pour recevoir la valeur pour le paramètre de la pompe à insuline (12), le processeur (40) pouvant aussi fonctionner pour déterminer si la régie est violée par la requête sur la base de la valeur pour le paramètre, et le processeur (40) pouvant aussi fonctionner pour exécuter la fonction en utilisant la valeur reçue pour le paramètre, si la requête n'est pas violée.

12. Dispositif de commande de pompe selon la revendication 11, dans lequel la règle limite la durée d'administration d'insuline à une durée maximale, et dans lequel la valeur pour le paramètre est la durée maximale en particulier dans lequel la règle limite un décalage avant le début d'administration d'insuline et dans lequel la valeur du paramètre est le décalage maximal.

13. Dispositif de commande de pompe selon la revendication 11 ou 12, comprenant en outre un dispositif d'émission, et dans lequel le dispositif d'émission peut fonctionner pour produire un message indiquant que la requête viole la règle, en particulier dans lequel le dispositif d'émission est un affichage qui peut fonctionner pour visuellement afficher le message.

14. Procédé permettant de faire fonctionner un système de traitement par insuline (10) qui comprend une pompe à insuline (12) et un dispositif de commande de pompe (18), dans lequel le dispositif de commande de pompe (18) est configuré pour communiquer avec la pompe à insuline (12) pour obtenir des valeurs de paramètre pour exécuter des fonctions, le procédé comprenant :
- la réception, par le dispositif de commande de pompe (18), d'une requête pour exécuter une fonction, dans lequel un processeur (40) du dispositif de commande de pompe (18) est configuré pour réaliser cette fonction, dans lequel un dispositif mémoire (42) du dispositif de commande de pompe (18) est configuré pour stocker un logiciel qui met en œuvre la fonction étant régie par une règle ayant un paramètre, la fonction étant liée à l'administration d'insuline par la pompe à insuline (12); dans lequel la règle est stockée dans le dispositif mémoire (42) du dispositif de commande de pompe (18) ou dans lequel la règle est stockée dans le dispositif mémoire (42) du dispositif de commande de pompe (18) et dans un dispositif mémoire (24) de la pompe à insuline (12) ;
- l'établissement d'une communication entre la pompe à insuline (12) et le dispositif de commande de pompe (18) ;
- la requête, par le dispositif de commande de pompe (18), d'une valeur pour le paramètre au dispositif mémoire (24) de la pompe à insuline (12) ;
- la réception, par le dispositif de commande de pompe (18), de la valeur pour le paramètre de la pompe à insuline (12) ;
- la détermination, par le processeur (40) du dispositif de commande de pompe (18), du fait que la règle est ou non violée par la requête sur la base de la valeur pour le paramètre ; et
- l'exécution, par le dispositif de commande de pompe (18), de la fonction utilisant la valeur reçue pour le paramètre, si la règle n'est pas violée.
